# EUROPÄISCHE PATENTSCHRIFT

(11) **EP 0 774 961 B1**
(45) Veröffentlichungstag und Bekanntmachung des Hinweises auf die Patenterteilung: **07.05.2003**
(21) Anmeldenummer: 95925732.0
(22) Anmeldetag: 27.07.1995
(51) Int. Cl.: A61K 31/135

(54) **VERWENDUNG VON SELEGILIN ZUR BEHANDLUNG VON EPILEPTISCHEN ERKRANKUNGEN**
USE OF SELEGILIN IN THE TREATMENT OF EPILECTIC CONDITIONS
UTILISATION DE SELEGILINE DANS LE TRAITEMENT D'AFFECTIONS EPILEPTIQUES

(30) Priorität: 11.08.1994 DE 4428444
(43) Veröffentlichungstag der Anmeldung: 28.05.1997
(73) Patentinhaber: VIATRIS GmbH & Co. KG, 60314 Frankfurt am Main (DE)
(72) Erfinder: LÖSCHER, Wolfgang, D-30559 Hannover (DE)
(74) Vertreter: Wibbelmann, Jobst, Dr., Dipl.-Chem.
(86) Internationale Anmeldenummer: DE9500981
(87) Internationale Veröffentlichungsnummer: WO96004897

(56) Entgegenhaltungen:
- EP-A- 0 614 663
- WO-A-92/17169
- THE JOURNAL OF PHARMACOLOGY AND EXPERIMENTAL THERAPEUTICS, Bd. 274, Nr. 1, 2.März 1995 Seiten 307-314, LÖSCHER, W. ET AL 'Anticonvulsant and antiepileptogenic effect of l-deprenyl (selegiline) in the kindling model of epilepsy'
- INDIAN JOURNAL OF EXPERIMENTAL BIOLOGY, Bd. 25, Nr. 11, 1987 Seiten 761-770, MUKHOPADHYAY, M. ET AL 'Neuropharmacological studies on selective monoamine oxidase A and B inhibitors'
- PHARMACOLOGY BIOCHEMISTRY AND BEHAVIOUR, Bd. 23, Nr. 5, 1985 Seiten 753-757, SPARKS, D.L. ET AL 'Combined inhibition of serotonin uptake and oxidative deamination attenuates audiogenic seizures in DBA/2J mice'

## Beschreibung

Die Erfindung betrifft die Verwendung von Selegilin (= L-N-(1-Phenyl-isopropyl)Nmethyl-N-propinylamin) oder dessen pharmazeutisch verwendbare Salze in Arzneimitteln mit antiepileptischer Wirkung.

Es ist bekannt, daß Selegilin ein Blocker der Monoaminoxydase vom Typ B ist. Dieses Enzym baut in Gliazellen monoaminerge Neurotransmitter (vor allem Dopamin) ab, was zur Beendigung der Wirkung von Dopamin führt. Durch die Blockade des Enzyms wird der Dopamin-Spiegel im Gehim angehoben. Aufgrund dieser Wirkung wird Selegilin als Zusatzbehandlung zusammen mit Levodopa in der Therapie der Parkinson-Erkrankung eingesetzt (beispielsweise unter dem Warenzeichen Deprenyl im Handel).

In der Literatur werden weitere Wirkungen des N-(1-Phenyl-isopropyl)N-methyl-N-propinylamins beziehungsweise der L-Form (Selegilin) beschrieben.
- In WO 92/17 169 A1 wird eine neuroprotektive Wirkung mit bisher nicht definiertem Wirkmechanismus beschrieben. Es wird daher als Neuroprotektivum (Verhinderung des Nervenzellverlustes bei Erkrankungen des ZNS, insbesondere der Parkinson-Krankheit, dem Himtrauma und dem Trauma des Rückenmarks) zur Anwendung vorgeschlagen.
- Weiterhin sind folgende Wirkungen von Selegilin mit oder ohne Kombinationspartner patentiert, für die jedoch keine eindeutigen Wirkmechanismen bekannt sind:
   Therapie von
   a) Immunsystem-Dysfunktionen;
   b) Cushing-Syndrom ;
   c) psychischen Entzugssymptomen nach Cocain, Alkohol und Opiat-Sucht;
   d) Alterserscheinungen ;
   e) Alzheimer-Erkrankung;
   f) psychischen Symptomen beim Tabak-Entzug;
   g) Schizophrenie;
   h) psychischen Symptomen beim prä-menstruellem Syndrom;
   i) Reisekrankheit (Kinetose);
   j) Bluthochdruck;
   k) Depression in Kombination mit anderen Präparaten
   (US 5 192 808, US 4 861 800, US 4 868 218, US 4 579 870, CA 1 322 530, WO 92/21333 A1, WO 91/18592 A1, WO 90/04387, WO 90/01928, WO 88/04552, EP 252 290 A1)
- Als weitere Anwendung ist die Markierung von Gliosen bei degenerativen Erkrankungen durch markiertes Selegilin als Marker von Gliazellen als Patent angemeldet (US 7 052 921, US 6 853 119). In dieser Indikation wird auch die Diagnose von gliotischen Zentren vor der chirurgischen Entfernung dieser Vemarbungen aus dem Gehim von Epilepsiepatienten erwähnt. Dies ist jedoch ausschließlich eine diagnostische Anwendung. Die Autoren stellen fest, daß es keinerlei Hinweise für eine pathogenetische Beteiligung von Monoaminoxydase B bei der Epilepsie gibt, Selegilin eignet sich nur zum Markieren der Gliazellen (Kumlien E. u.a. , Epilepsia, Vol.33, No. 4. 1992, 610 ff).

Zur Behandlung von Epilepsien sind eine Reihe von Antikonvulsiva auf dem Markt. Phenytoin und Carbamazepin sollen als wichtigte Vertreter genannt werden. Die bisher zur Therapie eingesetzten Antikonvulsiva führen zur Unterdrückung des Krampfes, haben aber keine Wirkung auf die Entwicklung des eigentlichen epileptischen Herdes.

Aufgabe der vorliegenden Erfindung ist es, Arzneimittel mit guten antikonvulsiven und antiepileptogenen Wirkungen bereitzustellen.

Es wurde überraschend gefunden, daß Selegilin eine starke antikonvulsive und antiepileptogene Wirkung zeigt. Die Schwelle für die Auslösung fokaler und generalisierter Krämpfe wurde ähnlich stark angehoben wie durch die Standard-Antikonvulsiva Carbamazepin und Phenytoin.
Die Krampfschwelle gilt als das wichtigste Merkmal einer antikonvulsiven Wirkung. Epilepsie ist eine Schwellenphänomen, und ein Krampf wird beim Menschen durch Überschreiten der Schwelle durch endogene oder exogene Reize ausgelöst. Medikamente der ersten Wahl zur Behandlung der Epilepsie erhöhen die Schwelle, Medikamente der zweiten Wahl beeinflussen oft nicht die Schwelle sondern reduzieren nur die Krampfschwere des einzelnen Anfalls.

Auch in einem klassischen Epilepsiemodell, dem maximalen Elektroschock, konnte eine Wirkung von Selegilin auf die Schwelle nachgewiesen werden.

Aufgrund der pharmakologischen Untersuchungen zeigt Selegilin eine ähnliche Wirkung wie Medikamente der 1. Wahl.

Bei epileptischen Patienten kommt es im Verlauf oft zu einem Fortschreiten der Erkrankung, die Anfälle werden stärker und der Patent spricht schlechter auf die Behandlung an.
Von besonderem Interesse ist daher, daß Selegilin auch in der Lage ist, die Entwicklung des Epilepsieherdes zu verlangsamen und damit das Fortschreiten der Erkrankung zu bremsen.

Die antiepileptische Wirkung des N-(1-Phenyl-isopropyl)N-methyl-N-propinylamin) ist streng stereospezifisch. Nur die L-Form (Selegilin) wirkt antiepileptisch.
Die D-Form ist unwirksam. Sie löst in Dosen, die bei der L-Form ohne Nebenwirkungen sind, starke amphetaminähnliche Nebenwirkungen aus.

### Pharmakologische Untersuchungen

Ausgangspunkt für die Untersuchung von Selegilin im Amygdala-Kindling als Modell der fokalen Epilepsie und als Modell der Epileptogenese waren die Erkenntnisse über die neuroprotektive Wirkung von Selegilin.

Im Amygdala-Kindling wird durch wiederholte sehr schwache Stimulation von einem Hirngebiet der Ratte über Tiefenelektroden ein epileptischer Herd induziert. Dieser Herd ist permanent und Tiere, die voll gekindelt sind, sind als epileptisch zu bezeichnen. In diesem Modell können nun Antikonvulsiva untersucht werden. Werden sie voll gekindelten Tieren gegeben und wird ein Krampf ausgelöst, dann kann die Schwelle für die Auslösung des Krampfes erhöht sein (es muß stärker stimuliert werden, um einen Krampf auszulösen) und der trotzdem ausgelöste Krampf kann schwächer sein.

In diesem Modell können jedoch auch Medikamente untersucht werden, die - unabhängig von ihrer Wirkung gegen den ausgelösten Krampf - die Entwicklung des epileptischen Herdes unterdrücken. Die antikonvulsive Wirkung ist unabhängig von der Wirkung gegen die Entwicklung des Epilepsieherdes.

### Daten zur antikonvulsiven Wirkung

Methode nach Freemann FG u.a., Brain Res. Bull 1981; 7; 629-633, modifiziert nach Rundfeldt C u.a., Neuropharmacology 1990; 29; 845-851.

Amygdala Kindling, Anhebung der Schwelle zur Auslösung von fokalen Krämpfen bei vollgekindelten Ratten:

| | Krampfschwelle Anhebung in % |
|---|---|
| **L-Form (Selegilin)** | |
| 5 mg/kg i. p. | 0 |
| 10 mg/kg i. p. | +130 - +250 |
| 20 mg/kg i. p. | +70 |
| 40 mg/kg i. p. | +41 |
| **D-Form** | |
| 10 mg/kg | -12 |

### Beeinflussung der Entwicklung des epileptischen Herdes im Kindling

Methode nach Racine R u.a., Electroencephalograph Clin Neurophysiol. 1975; 38; 355-365, siehe auch Silver JM u.a., Ann Neurol 1991; 29; 356-363.

Bei der Entstehung des epileptischen Herdes im Kindling kommt es zu plastischen Veränderungen im Gehirn. Für die volle Ausprägung des Epilepsieherdes sind epileptische Entladungen einer bestimmten Gesamtdauer notwendig.
Unter der Behandlung mit Selegilin (5 und 10 mg/kg, 1x/Tag) werden 35 % und 53 % mehr Stimulationen gebraucht, bis der erste generalisierte Krampf auftritt.
Zudem sind signifikant längere epileptische Entladungen notwendig, um den Herd zu etablieren (52 % und 117 %). Dies weist darauf hin, daß Selegilin die zur Etablierung des Herdes notwendigen plastischen Veränderungen des Gehirns verzögert.

Nach dem Absetzen der Behandlung ist die Dauer der epileptischen Entladungen, die bei den voll gekindelten Tieren ausgelöst werden könne, signifikant kürzer als bei Kotrolltieren.
Darin zeigt sich daß der epileptische Herd weniger stark ausgeprägt ist als bei der Kontrollgruppe.

Selegilin kann somit als hochspezifischer wirksamer Arzneistoff zur Behandlung von epileptischen Erkrankungen eingesetzt werden.
Dabei wird nicht nur der Krampf unterdrückt, sondern auch das Fortschreiten der Erkrankung verlangsamt.

Die erfindungsgemäße Verbindung und Verfahren zu ihrer Herstellung sind bekannt.

Die Verbindungen können in bekannter Weise in die üblichen Formulierungen überführt werden wie Tabletten, Kapseln, Dragees, Pillen, Granulate, Sirupe, Emulsionen, Suspensionen und Lösungen, unter Verwendung inerter, nichttoxischer, pharmazeutisch geeigneter Trägerstoffe oder Lösungsmittel.

Hierbei sollte die Tagesdosis an Selegilin bei oraler oder partenteraler Gabe 5 - 20 mg betragen.

Erforderlichenfalls kann von den genannten Mengen abgewichen werden und zwar in Abhängigkeit vom Körpergewicht und der speziellen Art des Applikationsweges.

## Patentansprüche

1. Verwendung von Selegilin oder dessen pharmazeutisch verwendbaren Salze zur Herstellung eines Arzneimittels zur Behandlung von epileptischen Erkrankungen.

2. Verwendung von Selegilin oder dessen pharmazeutisch verwendbaren Salze zur Herstellung eines Arzneimittels gemäß Anspruch 1 insbesondere zur Unterdrückung der Krämpfe und zur Verlangsamung des Fortschreitens der Erkrankung.

## Claims

1. Use of selegiline or pharmaceutically utilizable salts thereof for the production of a medicament for the treatment of epileptic disorders.

2. Use of selegiline or pharmaceutically utilizable salts thereof according to claim 1, in particular for suppressing the convulsions and for slowing down the progression of the disorder.

## Revendications

1. Utilisation de sélégiline ou de ses sels phannaceutiquement utilisables pour la préparation d'un médicament destiné au traitement de maladies épileptiques.

2. Utilisation de sélégiline ou de ses sels pharmaceutiquement utilisables pour la préparation d'un médicament selon la revendication 1, en particulier pour supprimer les crampes et pour ralentir la progression de la maladie.
